# EUROPEAN PATENT APPLICATION

(11) **EP 2 070 941 A1**
(43) Date of publication of application: **17.06.2009**
(21) Application number: 07076086.3
(22) Date of filing: 14.12.2007
(51) Int. Cl.: C07J 1/00, C07J 41/00

(54) **Stereoselective synthesis of selective estrogen receptor down-regulators**

(71) Applicant: Bayer Schering Pharma Aktiengesellschaft, 13353 Berlin (DE); AstraZeneca AB, 151 85 Södertälje (SE)
(72) Inventor: The designation of the inventor has not yet been filed

(57) **Abstract**

The present invention relates to stereoselective synthesis of 7-α-substituted estra-4-ene-3,17-diones. Such compounds are valuable intermediates in the synthesis of 7-α-substituted estra-1,3,5(10)-triene-3,17-diols.

Key step in this synthesis is reacting a 3-keto-4,6-estradiene with a Grignard reagent selected from the group consisting of a magnesium-halide alkyl halide having at least 3 carbon atoms, a magnesium-halide alkenyl halide having at least 3 carbon atoms, and a magnesium-halide alkynyl halide having at least 3 carbon atoms, in the presence of a catalyst system comprising CuI.

The obtained 7α-alkyl, alkenyl and alkynyl halides can be used for the synthesis of pharmaceutically actice 7-α-substituted estra-1,3,5(10)-triene-3,17-diols (antiestrogens).

## Description

### FIELD OF THE INVENTION

The present invention relates to stereoselective synthesis of 7-α-substituted estra-4-ene-3,17-diones. Such compounds are valuable intermediates in the synthesis of 7-α-substituted estra-1,3,5(10)-triene-3,17-diols. The present invention also relates to the synthesis of such 7-α-substituted estra-1,3,5(10)-triene-3,17-diols.

### BACKGROUND OF THE INVENTION

7α-substituted estra-1,3,5(10)-triene-3,17-diols are known to have anti-estrogenic activity, i.e. these compounds are estrogen antagonists, and are sometimes referred to as Selective Estrogen Receptor Down-regulators (SERDs). Such substances have already been described extensively.

For example, compounds that have an anti-estrogenic activity are known from EP 0 138 504. These are essentially estra-1,3,5(10)-triene derivatives, which are substituted in the 3-position with e.g. hydroxy or alkoxy, in the 17-β-position with hydroxy, and in the 17-α-position with hydrogen or alkyl. In the 7-α-position, these compounds also have an alkyl side chain that can be partially fluorinated and substituted with e.g. amido, amino, amine-N-oxide, oxy, sulfanyl, sulfinyl and/or sulfonyl groups.

In WO 99/33855, 11-β-halogen-7-α-substituted estra-1,3,5(10)-trienes are described that can have hydroxy groups in the 3-position and in the 17-position. The 7-α-side chain is a partially fluorinated, optionally unsaturated hydrocarbon chain that is interrupted by an amine-nitrogen atom or a sulfany, sulfinyl or sulfonyl group.

Other compounds are described in WO 98/07740. These are 7-α-substituted (ξ-aminoalkyl)-estra-1,3,5(10)-trienes. These compounds preferably have a hydroxy, methoxy or acetyloxy group in the 3-position and preferably a methyl or trifluoromethyl group in the 17-α-position and/or the 17-β-position. In the 11-p-position, a fluorine atom is preferably provided, and in the 7-α-position, an alkyl side chain that is at least partially fluorinated in the terminal position and that is interrupted by an amine-N atom and by a sulfanyl, sulfinyl or sulfonyl group is provided.

In WO 97/45441, 7-α-(5-methylaminopentyl)-estra-1,3,5(10)-trienes are disclosed that have a hydroxy group in the 3-position and in the 17-β-position. In the 17-α-position, a methyl or ethinyl group can be provided. The estratriene skeleton can also be substituted in the 2-position with a fluorine atom.

US 7,018,994 describes estra-1,3,5(10)-trien-3,17-diols with a 7-α-(ω-perfluoralkyl)-alkyl-side chain and methods for manufacturing these. US 7,018,994 is, however, silent regarding how to obtain a large 7-α/β-isomer ratio.

US 6,677,324 describes the synthesis of intermediates for producing estra-1,3,5(10)-trien-3,17-diols with a 7-α-(ω-perfluoralkyl)-alkyl side chain. The process of this patent, however, leads to products with a significant amount of the 7-β-isomer in addition to the 7-α-isomer. Hence, re-crystallisation or chromatography is necessary to isolate the desired 7-α-isomer.

A key step in the formation of 7-α-substituted estra-1,3,5(10)-triene-3,17-diols is the 1,6-addition of a Grignard reagent to the dien-system of estra-4,6-diene-3,17-diones shown in Scheme I.

While the use of short-chain Grignard reagents are known to be able to provide an α/β-isomer ratio in the order 8:1 and even reaching 10: 1, the literature contains no examples of the use of Grignard reagents with a carbon chain length of four or more, which provide a final 7-α/β ratio exceeding 4:1 in favour of the 7-α-isomer (see, e.g., Robert Bucourt et al. J. Biol. Chem. 1978;253(22);8221-8228 and John A. Katzenellenbogen. Steroids. 1993;58;157-169.). The yields of isolated 7-α-products are some times in the range of 40 to 50%, but are generally lower.

Clearly, there is a need for alternative methods for synthesising anti-estrogenic 7-α-substituted estra-1,3,5(10)-triene-3,17-diols with a high 7-α/β ratio, including methods for synthesising suitable intermediates to be used in the process.

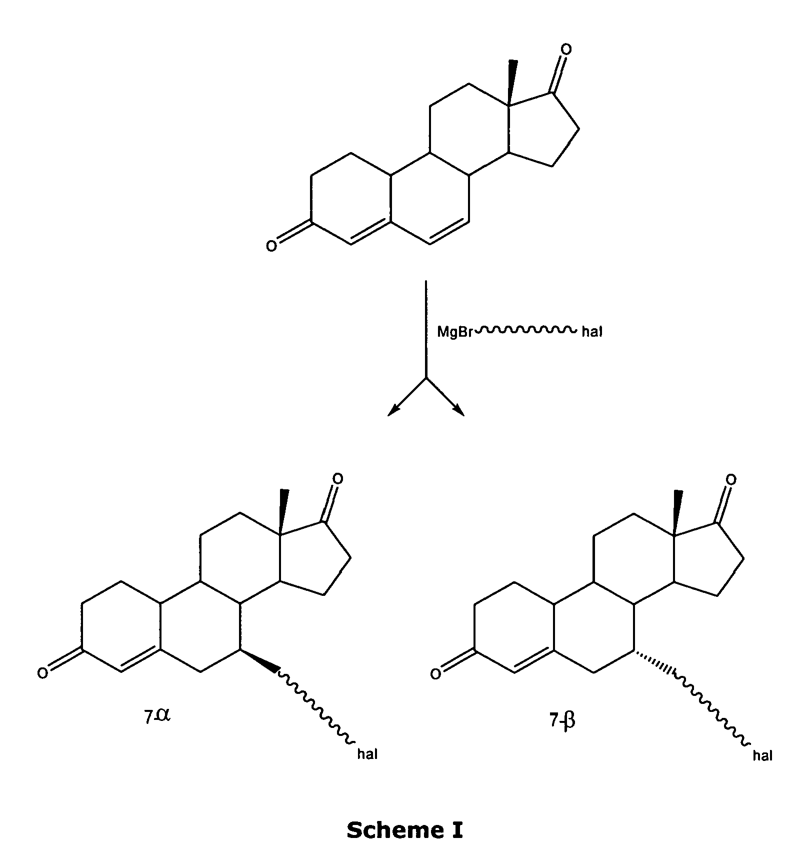

Accordingly, the object of the present invention is to provide a process for synthesising long-chain 7-α-substituted estra-4-ene-3,17-diones with a high 7-α/β ratio. Another object of the present invention is to provide a process for synthesising long-chain 7-α-substituted estra-1,3,5(10)-triene-3,17-diols with a high 7-α/β ratio.

### SUMMARY OF THE INVENTION

Accordingly, in a first aspect the present invention relates to a process for the manufacture of a compound of the general formula **I** 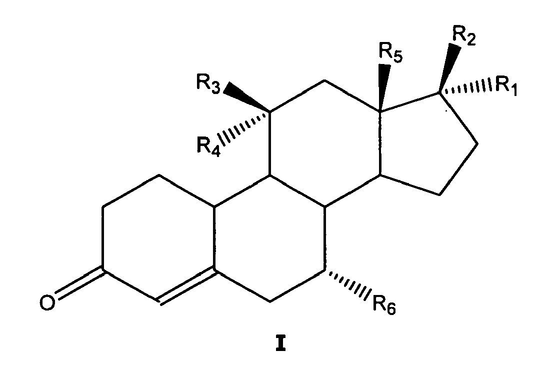 wherein
R₁ and R₂ taken together is O; or
R₁ is selected from the group consisting of H, C₁-C₆-alkyl, C₂-C₆-alkenyl and C₂-C₆-alkinyl; and
R₂ is OR_{A}, where R_{A} is H, C₁-C₆-alkyl or C₁-C₆-alkanoyl;
R₃ and R₄ are independently selected from the group consisting of H, F and OR_{B}, where R_{B} is H or an oxygen protecting group;
R₅ is CH₃ or CH₂-CH₃; and
R₆ is selected from the group consisting of an alkyl halide chain having at least 3 carbon atoms, an alkenyl halide chain having at least 3 carbon atoms, and an alkynyl halide chain having at least 3 carbon atoms;
said method comprising the step of reacting a compound of the general formula **II** 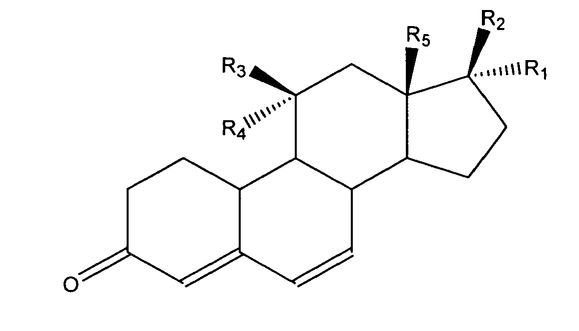 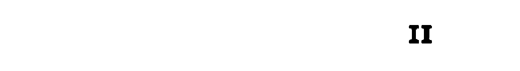 wherein R₁, R₂, R₃, R₄ and R₅ are as defined above, with a Grignard reagent selected from the group consisting of a magnesium-halide alkyl halide having at least 3 carbon atoms, a magnesium-halide alkenyl halide having at least 3 carbon atoms, and a magnesium-halide alkynyl halide having at least 3 carbon atoms, in the presence of a catalyst system comprising CuI.

In a further aspect the present invention relates to a process for the manufacture of a compound of the general formula **III** 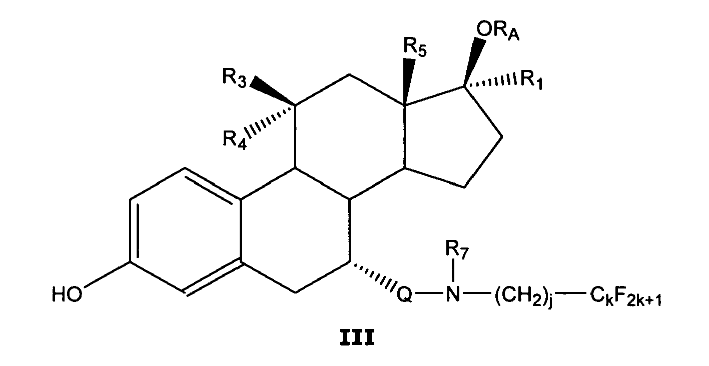 wherein
R₁ is selected from the group consisting of H, C₁-C₆-alkyl, C₂-C₆-alkenyl and C₂-C₆-alkinyl; and
R_{A} is H, C₁-C₆-alkyl or C₁-C₆-alkanoyl;
R₃ and R₄ are independently selected from the group consisting of H, F and OR_{B} where R_{B} is H or an oxygen protecting group;
R₅ is CH₃ or CH₂-CH₃;
Q is an alkyl chain having at least 3 carbon atoms, an alkenyl chain having at least 3 carbon atoms, or an alkynyl chain having at least 3 carbon atoms;
R₇ is H or C₁-C₄-alkyl;
j is an integer in the range of from 1 to 10; and
k is an integer in the range of from 1 to 5;
said process comprising the steps of
(i) reacting a compound of the general formula **IIa** 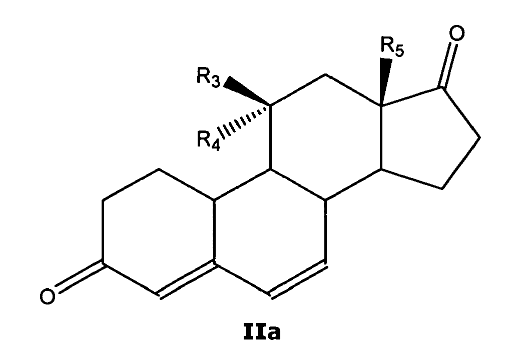 wherein R₃, R₄ and R₅ are as defined above, with a Grignard reagent selected from the group consisting of a magnesium-halide alkyl halide having at least 3 carbon atoms, a magnesium-halide alkenyl halide having at least 3 carbon atoms, and a magnesium-halide alkynyl halide having at least 3 carbon atoms, in the presence of a catalyst system comprising CuI, to obtain a compound of the general formula **Ia** 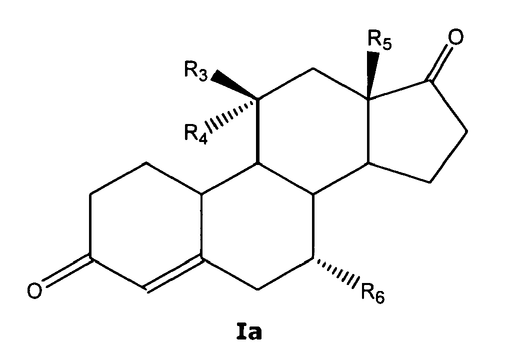 wherein R₃, R₄ and R₅ are as defined above, and R₆ is selected from the group consisting of an alkyl halide chain having at least 3 carbon atoms, an alkenyl halide chain having at least 3 carbon atoms, and an alkynyl halide chain having at least 3 carbon atoms; and
(ii) converting the compound of the general formula **Ia** to the compound of the general formula **III.**

### DETAILED DESCRIPTION OF THE INVENTION

In the present context, the term "C₁-C₆-alkyl" is intended to mean a linear or branched saturated hydrocarbon chain having from one to six carbon atoms, such as methyl, ethyl, n-propyl, isopropyl, n-butyl, isobutyl, sec-butyl, tert-butyl, n-pentyl, isopentyl, neopentyl or n-hexyl. Likewise, the term "C₁-C₄-alkyl" is intended to mean a linear or branched saturated hydrocarbon chain having from one to four carbon atoms, such as methyl, ethyl, n-propyl, isopropyl, n-butyl, isobutyl, sec-butyl or tert-butyl.

In the present context, the term "C₁-C₆-alkanoyl" is intended to mean a formyl-, group or a group of the partial formula -C(O)-C₁-C₅-alkyl wherein C₁-C₅-alkyl stands for a linear or branched saturated hydrocarbon chain having from one to five carbon atoms, such as methyl, ethyl, n-propyl, isopropyl, n-butyl, isobutyl, sec-butyl, tert-butyl, n-pentyl, isopentyl, neopentyl.

In the present context, the term "C₂-C₆-alkenyl" is intended to mean a linear or branched hydrocarbon chain having from two to six carbon atoms and containing one or more double bonds. Illustrative examples of C₂-C₆-alkenyl groups include allyl, homo-allyl, vinyl, crotyl, butenyl, pentenyl and hexenyl.

In the present context, the term "halogen" or "halide" covers F, Cl, Br and I.

When used herein, the term "alkyl halide chain having at least 3 carbon atoms" is intended to mean a linear or branched saturated hydrocarbon chain having at least three carbon atoms, such as 3-10, 3-7 or 4-6 carbon atoms, where the hydrocarbon chain is substituted with at least one halogen atom. Preferably, the hydrocarbon chain is substituted with only one halogen atom, in particular Cl. Moreover, the halogen atom is preferably substituted at the terminal methyl group, i.e. the alkyl halide is preferably a primary (1°) alkyl halide. Likewise, the term "magnesium-halide alkyl halide having at least 3 carbon atoms" is intended to mean an alkyl halide chain as defined above, which has been further substituted with a magnesium halide group, in particular MgBr.

When used herein, the term "alkenyl halide chain having at least 3 carbon atoms" is intended to mean a linear or branched hydrocarbon chain having at least three carbon atoms, such as 3-10, 3-7 or 4-6 carbon atoms, and containing one or more double bonds and where the hydrocarbon chain is substituted with at least one halogen atom. Preferably, the hydrocarbon chain is substituted with only one halogen atom, in particular Cl. Moreover, the halogen atom is preferably substituted at a terminal methyl group, i.e. the alkenyl halide is preferably a primary (1°) alkenyl halide. Likewise, the term "magnesium-halide alkenyl halide having at least 3 carbon atoms" is intended to mean an alkenyl halide chain as defined above, which has been further substituted with a magnesium halide group, in particular MgBr.

When used herein, the term "alkynyl halide chain having at least 3 carbon atoms" is intended to mean a linear or branched hydrocarbon chain having at least three carbon atoms, such as 3-10, 3-7 or 4-6 carbon atoms, and containing one or more triple bonds and where the hydrocarbon chain is substituted with at least one halogen atom. Preferably, the hydrocarbon chain is substituted with only one halogen atom, in particular Cl. Moreover, the halogen atom is preferably substituted at a terminal methyl group, i.e. the alkynyl halide is preferably a primary (1°) alkynyl halide. Likewise, the term "magnesium-halide alkynyl halide having at least 3 carbon atoms" is intended to mean an alkynyl halide chain as defined above, which has been further substituted with a magnesium halide group, in particular MgBr.

In the present context, the term "tri-C₁-C₆-alkylsilyl halide" is intended to mean compounds of the general formula (R₈)(R₉)(R₁₀)Si-Y, wherein each of R₈, R₉ and R₁₀ are C₁-C₆-alkyl, preferably C₁-C₄-alkyl, and wherein Y is halogen, in particular chlorine. Specific examples of tri-C₁-C₆-alkylsilyl halides include tert-butyl-dimethylsilyl chloride and trimethylsilyl chloride.

Herein, the term "oxygen protecting group" is used within its common meaning and such oxygen protecting groups are well-known to the person skilled in the art. Commonly used oxygen protecting groups include, but is not limited to, methyl, benzyl, methoxy- or dimethoxybenzyl, silyl, trialkylsilyl, acyl such as acetyl or formyl, alkylsulfonyl, arylsulfonyl, and the like.

As indicated above, one aspect of the present invention is directed to a process for the manufacture of a compound of the general formula **I** 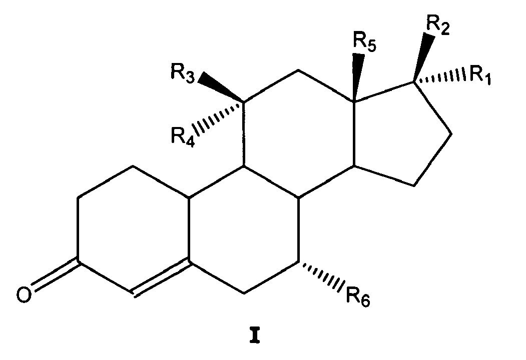 wherein
R₁ and R₂ taken together is O; or
R₁ is selected from the group consisting of H, C₁-C₆-alkyl, C₂-C₆-alkenyl and C₂-C₆-alkinyl; and
R₂ is OR_{A}, where R_{A} is H, C₁-C₆-alkyl or C₁-C₆-alkanoyl;
R₃ and R₄ are independently selected from the group consisting of H, F and OR_{B}, where R_{B} is H or an oxygen protecting group;
R₅ is CH₃ or CH₂-CH₃; and
R₆ is selected from the group consisting of an alkyl halide chain having at least 3 carbon atoms, an alkenyl halide chain having at least 3 carbon atoms, and an alkynyl halide chain having at least 3 carbon atoms;
said method comprising the step of reacting a compound of the general formula **II** 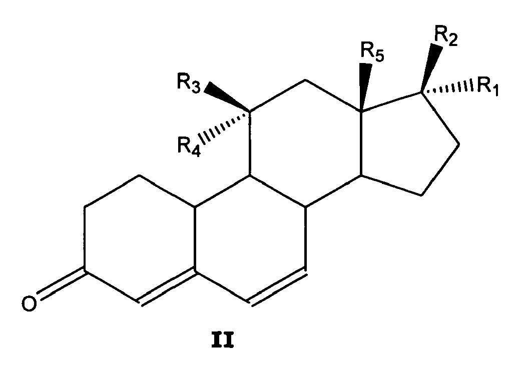 wherein R₁, R₂, R₃, R₄ and R₅ are as defined above, with a Grignard reagent selected from the group consisting of a magnesium-halide alkyl halide having at least 3 carbon atoms, a magnesium-halide alkenyl halide having at least 3 carbon atoms, and a magnesium-halide alkynyl halide having at least 3 carbon atoms, in the presence of a catalyst system comprising CuI.

The present inventors have surprisingly realised that by employing the reaction conditions described herein, a high stereoselectivity with respect to the R₆ substituent in the 7-position can be obtained, i.e. a high α/β ratio in the 7-position can be obtained. Accordingly, in an interesting embodiment of the invention, the process is carried out under such conditions that the molar ratio between the compound of the general formula **I** (the 7-α-isomer) and a by-product of the general formula **I*** (the 7-β-isomer) 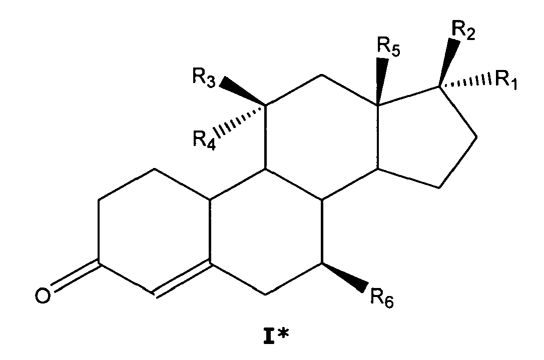 formed during the reaction is more than 1:1. Preferably, the molar ratio between the compound of the general formula **I** and the by-product of the general formula **I*** is more than 1.25: 1, such as more than 1.5: 1, e.g. more than 1.75: 1, more preferably more than 2:1, such as more than 3:1, e.g. more than 5:1, even more preferably more than 6: 1, such as more than 8: 1, e.g. more than 10: 1. Without being limited by any theory, it is contemplated that, in particular, application of the correct temperature is of importance in connection with achieving a high α/β ratio.

Concerning the substituent at position 17, R₁ may be selected from the group consisting of H, C₁-C₆-alkyl and C₂-C₆-alkenyl. R₂ is H or OR_{A}, where R_{A} is H or C₁-C₆-alkyl. However, in a preferred embodiment of the invention R₁ and R₂, when taken together, is O, i.e. the fused cyclopentyl ring (conventionally referred to as the D ring) of the compounds of the general formulae I and II has the following structure:

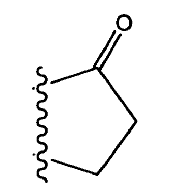

Concerning the substituents at position 11, R₃ and R₄ may be independently selected from the group consisting of H, F and OR_{B}, where R_{B} is H or an oxygen protecting group. In a preferred embodiment of the invention, R₃ is F and R₄ is H.

With respect to the substituent at position 13, R₅ may be methyl or ethyl (CH₃ or CH₂-CH₃). In a preferred embodiment of the invention, R₅ is methyl (CH₃).

R₆ (the substituent at position 7) may, as discussed above, be selected from the group consisting of an alkyl halide chain having at least 3 carbon atoms, an alkenyl halide chain having at least 3 carbon atoms, and an alkynyl halide chain having at least 3 carbon atoms. Preferably, R₆ is an alkyl halide chain having at least 3 carbon atoms, such as 3-10, 3-7 or 4-6 carbon atoms. Thus, in an interesting embodiment of the invention, R₆ may be represented by the general formula -(CH₂)ₙ-CH₂X, wherein n is an integer in the range of from 2 to 9, and wherein X is F or Cl, preferably Cl. In general, it is preferred that n is an integer in the range of from 2 to 6, more preferably an integer in the range of from 3 to 5, in particular an integer of 4. Accordingly, in a highly preferred embodiment of the invention, R₆ has the chemical structure (CH₂)₄-CH₂Cl, i.e. R₆ is a 1-chloro-n-pentyl group.

As will be understood by the skilled person, the preferred structures of R₆ correspond to the preferred structures of the Grignard reagent. Thus, the Grignard reagent may, as also discussed above, be selected from the group consisting of a magnesium-halide alkyl halide having at least 3 carbon atoms, a magnesium-halide alkenyl halide having at least 3 carbon atoms, and a magnesium-halide alkynyl halide having at least 3 carbon atoms. Preferably, the Grignard reagent has at the most 10 carbon atoms. In a preferred embodiment of the invention, the Grignard reagent is a magnesium-halide alkyl halide having 3 to 10 carbon atom, such as 4 to 10 carbon atoms, e.g. 4 to 8 carbon atoms, more preferably 4 to 6 carbon atoms, in particular 5 carbon atoms. The magnesium-halide alkyl halide is typically a magnesium-halide alkyl fluoride or a magnesium-halide alkyl chloride. In particular, the magnesium-halide alkyl chloride is preferred. Moreover, the magnesium-halide alkyl halide is preferably a magnesium-bromide alkyl halide, in particular a magnesium-bromide alkyl chloride. Stated differently, in a preferred embodiment of the invention, the Grignard reagent may be represented by the general formula BrMg-(CH₂)ₘ-CH₂X, wherein m is an integer in the range of from 2 to 9, and wherein X is F or Cl, preferably Cl. In general, it is preferred that m is an integer in the range of from 2 to 6, more preferably an integer in the range of from 3 to 5, in particular an integer of 4. Accordingly, in a highly preferred embodiment of the invention, the Grignard reagent has the chemical structure BrMg-CH₂-CH₂-CH₂-CH₂-CH₂Cl.

Accordingly, based on the above disclosure, it will be understood that in a highly preferred embodiment of the invention the compound of the general formula I has the following chemical structure:

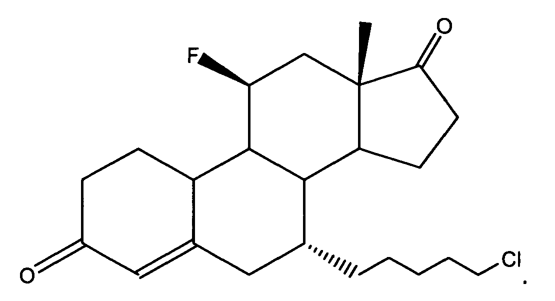

Likewise, it it will be understood that in a highly preferred embodiment of the invention the compound of the general formula **II** has the following chemical structure:

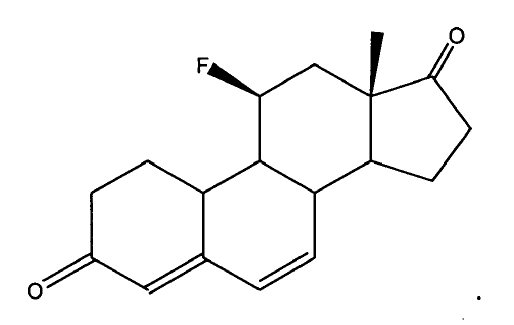

The process of converting a compound of the general formula **II** into a compound of the general formula **I** takes place in the presence of a catalyst system comprising CuI. In a highly preferred embodiment of the invention the catalyst system further comprises LiCl. In this embodiment, the ratio between CuI and LiCl present in the catalyst system is chosen so that the molar ratio between CuI and LiCl is typically in the range of from 1:1 to 1:4, such as in the range of from 1:1.5 to 1:2.5. In the most preferred embodiment, the molar ratio between CuI and LiCl is about 1:2.

Independently of the presence of LiCl in the catalyst system, the reactant, i.e. the compound of the general formula **II** is typically used in excess relative to the amount of CuI. Accordingly, the ratio between the compound of the general formula **II** and CuI is typically in the range of from 1:1 to 20: 1, such as in the range of from 1:1 to 1:10, e.g. in the range of from 1:1 to 1:5. In a preferred embodiment of the invention, the ratio between the compound of the general formula **II** and CuI is in the range of from 1.5:1 to 3:1, more preferably about 2:1.

In a preferred embodiment of the invention, the reaction is carried out in the presence of a tri-C₁-C₆-alkylsilyl halide, such as trimethylsilyl chloride or tert-butyl-dimethylsilyl chloride, in particular trimethylsilyl chloride. The tri-C₁-C₆-alkylsilyl halide may be added to the reaction mixture before, after or simultaneously with the addition of the CuI-containing catalyst system, but is preferably added to the reaction mixture after addition of the catalyst system comprising CuI. When added to the reaction, the tri-C₁-C₆-alkylsilyl halide is typically used in excess relative to the compound of the general formula **II**. Thus, in a typical embodiment of the invention, the molar ratio between the tri-C₁-C₆-alkylsilyl halide and the compound of the general formula **II** is in the range of range of from 1:1 to 10: 1, such as in the range of from 2:1 to 8: 1, preferably in the range of from 4:1 to 6: 1, more preferably about 5: 1.

Concerning the Grignard reagent, this reagent is also typically used in excess relative to the compound of the general formula **II**. Thus, in a typical embodiment of the invention, the molar ratio between the Grignard reagent and the compound of the general formula **II** is in the range of from 1:1 to 5: 1, such as in the range of from 1:1 to 3:1, preferably about 2:1.

The reaction is typically carried out a temperature below the freezing point, i.e. the reaction is typically carried out at a temperature in the range of from -80 to 0°C, such as in the range of from -80 to -20°C, e.g. in the range of from -80 to -40°C. In a highly preferred embodiment of the invention, the reaction is carried out at a temperature in the range of from -75 to -55°C, more preferably at a temperature of about -65°C.

The reaction solvent is preferably a polar and aprotic solvent, such as a polar and aprotic solvent selected from the group consisting of tetrahydrofuran (THF), 1,4-dioxane, 2-methyl THF, diethyl ether, *tert*-butylmethyl ether and mixtures thereof. Preferably, the reaction is carried out in THF.

As indicated above, the compounds of the general formula **I** are useful intermediates in the synthesis of antiestrogenic estra-1,3,5(10)-trien-3,17-diols with a 7α-(perfluoralkyl)-N-alkyl-side chain. Accordingly, a second aspect of the present invention is directed to a process for the manufacture of a compound of the general formula **III** 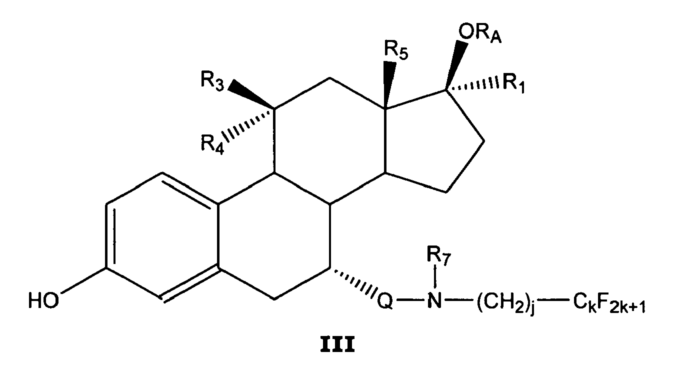 wherein
R₁ is selected from the group consisting of H, C₁-C₆-alkyl, C₂-C₆-alkenyl and C₂-C₆-alkinyl; and
R_{A} is H, C₁-C₆-alkyl or C₁-C₆-alkanoyl;
R₃ and R₄ are independently selected from the group consisting of H, F and OR_{B} where R_{B} is H or an oxygen protecting group;
R₅ is CH₃ or CH₂-CH₃;
Q is an alkyl chain having at least 3 carbon atoms, an alkenyl chain having at least 3 carbon atoms, or an alkynyl chain having at least 3 carbon atoms;
R₇ is H or C₁-C₄-alkyl;
j is an integer in the range of from 1 to 10; and
k is an integer in the range of from 1 to 5;
said process comprising the steps of
(i) reacting a compound of the general formula **IIa** 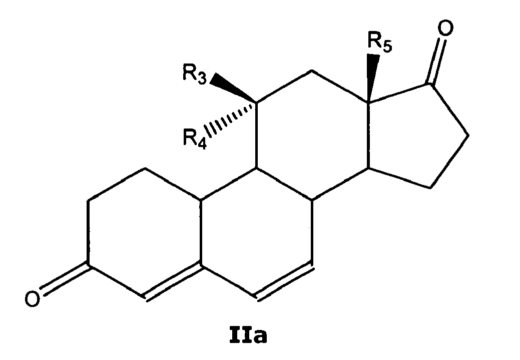 wherein R₃, R₄ and R₅ are as defined above, with a Grignard reagent selected from the group consisting of a magnesium-halide alkyl halide having at least 3 carbon atoms, a magnesium-halide alkenyl halide having at least 3 carbon atoms, and a magnesium-halide alkynyl halide having at least 3 carbon atoms, in the presence of a catalyst system comprising CuI, to obtain a compound of the general formula **Ia** 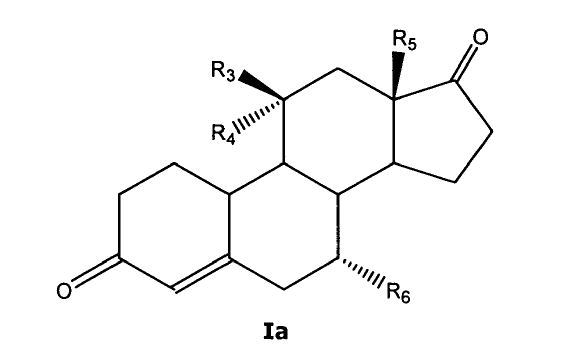 wherein R₃, R₄ and R₅ are as defined above, and R₆ is selected from the group consisting of an alkyl halide chain having at least 3 carbon atoms, an alkenyl halide chain having at least 3 carbon atoms, and an alkynyl halide chain having at least 3 carbon atoms; and
(ii) converting the compound of the general formula **Ia** to the compound of the general formula **III**.

All statements made above concerning preferred embodiments, such as with regard to reaction conditions, substituents, etc. apply *mutatis mutandis* to this aspect of the present invention. Accordingly, R₁ may be selected from the group consisting of H, C₁-C₆-alkyl, C₂-C₆-alkenyl and C₂-C₆-alkinyl. Preferably, R₁ is CH₃. R_{A} may be H, C₁-C₆-alkyl or C₁-C₆-alkanoyl, but is preferably H. R₃ and R₄ may be independently selected from the group consisting of H, F and OR_{B}, where R_{B} is H or an oxygen protecting group. In a preferred embodiment of the invention, R₃ is F and R₄ is H. R₅ may be CH₃ or CH₂-CH₃. In a preferred embodiment of the invention, R₅ is CH₃.

The preferred structures of Q and R₆ are, as will be understood by the skilled person, closely related. R₆ is preferably a group as described above in connection with the first aspect of the present invention which, in turn, has the consequence that Q may be selected from the group consisting of an alkyl chain having at the most 10 carbon atoms, an alkenyl chain having at the most 10 carbon atoms or an alkynyl chain having at the most 10 carbon atoms. Preferably Q is an alkyl chain having 3-10, 3-7 or 4-6 carbon atoms. Thus, in an interesting embodiment of the invention, Q may be represented by the formula (CH₂)ᵢ wherein i is an integer in the range of from 3 to 10, such as an integer in the range of from 3 to 7, e.g. 3, 4, 5, 6 or 7, preferably an integer in the range of from 4 to 6, such as 4, 5 or 6. Most preferably, i is an integer of 5, i.e. Q has the chemical structure CH₂-CH₂-CH₂-CH₂-CH₂-.

Concerning step (ii) above, i.e. the conversion of a compound of the general formula **Ia** to a compound of the general formula **III**, this may be achieved by a number of standard reaction step, which are known to the person skilled in the art and described in WO 03/045972 and US 7,018,994. Accordingly, in one embodiment of the invention, step (ii) above comprises the following steps:
(iia) converting a compound of the general formula **Ia** to a compound of the general formula **IV** 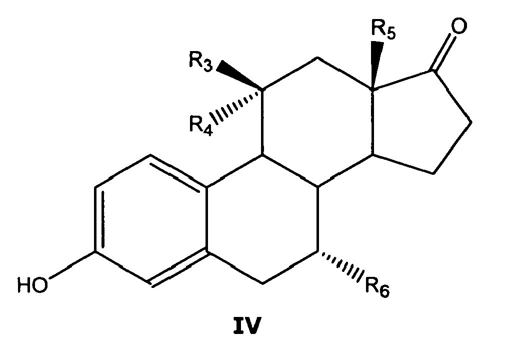
(iib) converting a compound of the general formula **IV** to a compound of the general formula **V** 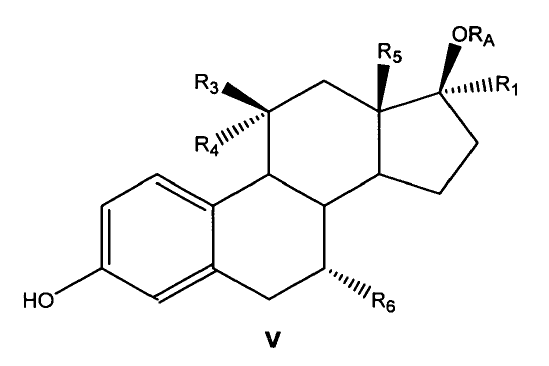
(iic) converting a compound of the general formula **V** to the compound of the general formula **III**, wherein R_{A}, R₁, R₃, R₄, R₅ and R₆ are as previously defined herein.

Concerning steps (iia) and (iib) these are accomplished according to procedures known to those skilled in the art,, e.g. (iia) as described in WO97/45441, WO98/07740 and WO99/33855, especially according to Example 29b of WO98/07740 and (iib) as described in WO03/045972, variant 2.2.a), page 27.

Step (iic) is conveniently carried out, e. g. analogously to process variant 2.2.b) page 27 of WO98/07740, by reacting a compound of the general formula **V** with a compound of the general formula **VI**

NHR₇-(CH₂)ⱼ-CₖF₂ₖ₊₁ VI

wherein
R₇ is H or C₁-C₄-alkyl;
j is an integer in the range of from 1 to 10; and
k is an integer in the range of from 1 to 5

Concerning R₇, this substituent may be H or C₁-C₄-alkyl. In a preferred embodiment of the invention, R₇ is H, CH₃ or CH₂-CH₃, in particular CH₃.

j is an integer in the range of from 1 to 10, preferably an integer in the range of from 5 to 9, such as 5, 6, 7, 8 or 9. More preferably, j is an integer in the range of from 6 to 8, in particular 7.

k is an integer in the range of from 1 to 5, such as 1, 2 or 3. Preferably, k is 2.

Accordingly, based on the above disclosure, it will be understood that in a highly preferred embodiment of the invention the compounds of the general formulae **III** and **VI** have the following chemical structures: 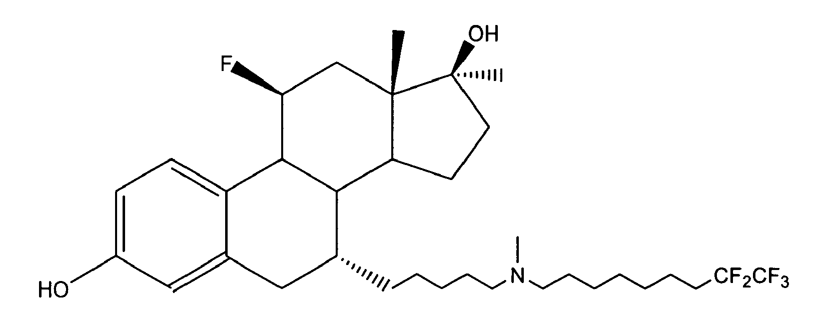 and

NH(CH₃)-(CH₂)₇-CF₂-CF₃.

### EXAMPLES

### 7α-(5-Chloropentyl)-11-β-fluoroestr-4-ene-3,17-dione

3.38 g (138.72 mmol) magnesium chips were suspended in 100 ml THF. 1.86 ml of a 20% solution of DIBAH in toluene was added (20°C) to activate the magnesium. A solution of 25.74 g (138.72 mmol) 1-bromo-5-chloropentane in 80 ml of THF was prepared separately. Approx. 10% of this solution was added drop-wise to the magnesium chips under vigorous stirring. Following the initiation of the reaction (duration approx. 5 to 10 minutes, may be determined by development of heat), the remaining bromide solution was continuously added over 30 minutes at 25°C to 30°C within 30 minutes. Subsequently, the mixture was stirred for 1.5 h at 23°C (whereby the magnesium was completely dissolved).

In order to carry out the 1,6 addition, 20 g (69.36 mmol) of 11-β-fluoroestra-4,6-diene-3,17-dione was suspended in 180 ml THF. 2.94 g (69.36 mmol) of LiCl and 6.6 g (34.68 mmol) of Cul were mixed with 25 ml of THF and stirred for 1 hour at room temperature. The steroid solution was cooled to -10°C and the catalyst solution was added to the steroid solution. Subsequently, 44 ml (346.79 mmol) of trimethylsilyl chloride was added, and the mixture was cooled to -70°C to -60°C. 80% of the Grignard solution prepared above was added drop-wise to this solution over 3 hours.

After completing the reaction, the reaction mixture was quenched by adding 40 ml of 2M HCl and the reaction mixture was heated to room temperature. Subsequently, 80 ml of water was added, and the layers were separated.

The aqueous phase was extracted once with 100 ml of toluene, and the combined organic phases were washed once with a mixture of 70 ml of an ammonia solution and 70 ml of water. Subsequently, the organic phase was washed twice with a solution of 18 g of ammonium chloride and 16 ml of ammonia solution in 100 ml of water.

The solvent was subsequently evaporated, and the residue was separated chromatographically to determine the yield. 19,2 g (70% of theory) of 7α-(5-Chloropentyl)-11-β-fluoroestr-4-ene-3,17-dione was obtained. Likewise, 1,9 g (6,9% of theory) of 7β-(5-Chloropentyl)-11-β-fluoroestr-4-ene-3,17-dione was obtained. Thus, the obtained ratio between the α and the β isomer was 10:1.

## Claims

1. A process for the manufacture of a compound of the general formula I 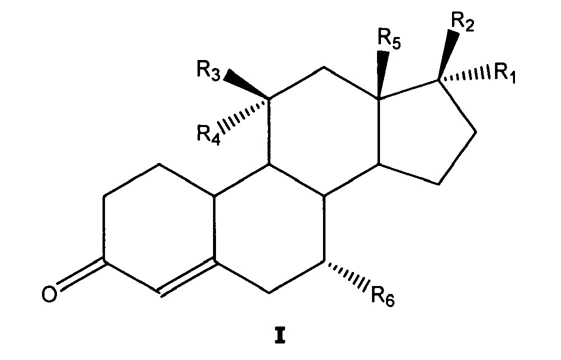 wherein
R₁ and R₂ taken together is O; or
R₁ is selected from the group consisting of H, C₁-C₆-alkyl,C₂-C₆-alkenyl and C₂-C₆-alkinyl; and
R₂ is OR_{A}, where R_{A} is H, C₁-C₆-alkyl or C₁-C₆-alkanoyl;
R₃ and R₄ are independently selected from the group consisting of H, F and OR_{B}, where R_{B} is H or an oxygen protecting group;
R₅ is CH₃ or CH₂-CH₃; and
R₆ is selected from the group consisting of an alkyl halide chain having at least 3 carbon atoms, an alkenyl halide chain having at least 3 carbon atoms, and an alkynyl halide chain having at least 3 carbon atoms;
said method comprising the step of reacting a compound of the general formula **II** 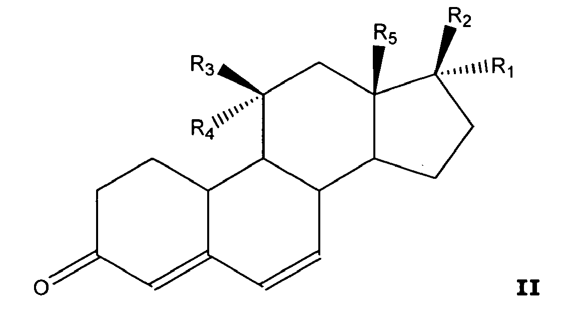 wherein R₁, R₂, R₃, R₄ and R₅ are as defined above, with a Grignard reagent selected from the group consisting of a magnesium-halide alkyl halide having at least 3 carbon atoms, a magnesium-halide alkenyl halide having at least 3 carbon atoms, and a magnesium-halide alkynyl halide having at least 3 carbon atoms, in the presence of a catalyst system comprising CuI.

2. The process according to claim 1, wherein said magnesium-halide alkyl halide, said magnesium-halide alkenyl halide or said magnesium-halide alkynyl halide has at the most 10 carbon atoms.

3. The process according to claim 1 or 2, wherein said catalyst system further comprises LiCl.

4. The process according to claim 3, wherein the molar ratio between CuI and LiCl is in the range of from 1:1 to 1:4, such as in the range of from 1:1.5 to 1:2.5, preferably about 1:2.

5. The process according to any of the preceding claims, wherein the molar ratio between the compound of the general formula II and CuI is in the range of from 1:1 to 20:1, such as in the range of from 1:1 to 1:10, e.g. in the range of from 1:1 to 1:5, preferably in the range of from 1.5:1 to 3:1, more preferably about 2:1.

6. The process according to any of the preceding claims, wherein said Grignard reagent is a magnesium-halide alkyl halide having 3 to 10 carbon atoms, such as 4 to 10 carbon atoms, e.g. 4 to 8 carbon atoms, preferably 4 to 6 carbon atoms, more preferably 5 carbon atoms.

7. The process according to claim 6, wherein said magnesium-halide alkyl halide is a magnesium-halide alkyl chloride or a magnesium-halide alkyl fluoride, preferably a magnesium-halide alkyl chloride.

8. The process according to any of the preceding claims, wherein said magnesium-halide alkyl halide is a magnesium-bromide alkyl halide.

9. The process according to claim 8, wherein said magnesium-bromide alkyl halide has the general formula BrMg-(CH₂)ₘ-CH₂X, wherein m is an integer in the range of from 2 to 9, and wherein X is F or Cl, preferably Cl.

10. The process according to claim 9, wherein m is an integer in the range of from 2 to 6, preferably an integer in the range of from 3 to 5, more preferably an integer of 4.

11. The process according to any of the preceding claims, wherein R₆ has the general formula -(CH₂)ₙ-CH₂X, wherein n is an integer in the range of from 2 to 9, and wherein X is F or Cl, preferably Cl.

12. The process according to claim 11, wherein n is an integer in the range of from 2 to 6, preferably an integer in the range of from 3 to 5, more preferably an integer of 4.

13. The process according to any of the preceding claims, wherein the reaction is carried out in the presence of a tri-C₁-C₆-alkylsilyl halide.

14. The process according to claim 13, wherein said tri-C₁-C₆-alkylsilyl halide is tri-methylsilyl chloride or *tert*-butyl-dimethylsilyl chloride, preferably tri-methylsilyl chloride.

15. The process according to claim 13 or 14, wherein said tri-C₁-C₆-alkylsilyl halide is added to the reaction mixture after addition of the catalyst system comprising CuI.

16. The process according to any of claims 13-15, wherein the molar ratio between said tri-C₁-C₆-alkylsilyl halide and the compound of the general formula **II** is in the range of from 1:1 to 10: 1, such as in the range of from 2:1 to 8: 1, e.g. in the range of from 4:1 to 6:1, preferably about 5:1.

17. The process according to any of the preceding claims, wherein the molar ratio between the Grignard reagent and the compound of the general formula II is in the range of from 1:1 to 5:1, such as in the range of from 1:1 to 3:1, preferably about 2: 1.

18. The process according to any of the preceding claims, wherein said reaction is carried out at a temperature in the range of from -80 to 0°C, such as in the range of from -80 to -20°C, e.g. in the range of from -80 to -40°C, preferably in the range of from -75 to -55°C, more preferably at about -65°C.

19. The process according to any of the preceding claims, wherein said reaction is carried out in a polar and aprotic solvent.

20. The process according to claim 19, wherein said polar and aprotic solvent is selected from the group consisting of tetrahydrofuran (THF), 1,4-dioxane, 2-methyl THF, diethyl ether, *tert*-butylmethyl ether and mixtures thereof, preferably THF.

21. The process according to any of the preceding claims, wherein the molar ratio between the compound of the general formula **I** and a by-product of the general formula **I*** 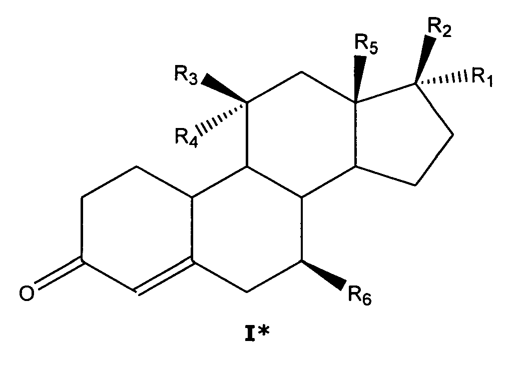 formed during the reaction, wherein R₁, R₂, R₃, R₄, R₅ and R₆ are as defined in any of claims 1-20, is more than 1:1.

22. The process according to claim 21, wherein the molar ratio between the compound of the general formula **I** and the by-product of the general formula **I*** is more than 1.25:1, such as more than 1.5:1, e.g. more than 1.75:1, preferably more than 2: 1, such as more than 3: 1, e.g. more than 5: 1, more preferably more than 6: 1, such as more than 8: 1, e.g. more than 10: 1.

23. The process according to any of the preceding claims, wherein R₁ and R₂ taken together is O.

24. The process according to any of the preceding claims, wherein R₃ is F.

25. The process according to any of the preceding claims, wherein R₄ is H.

26. The process according to any of the preceding claims, wherein R₅ is CH₃.

27. The process according to any of the preceding claims, wherein R₆ is (CH₂)₄-CH₂Cl.

28. The process according to any of the preceding claims, wherein the compound of the general formula **I** has the following chemical structure

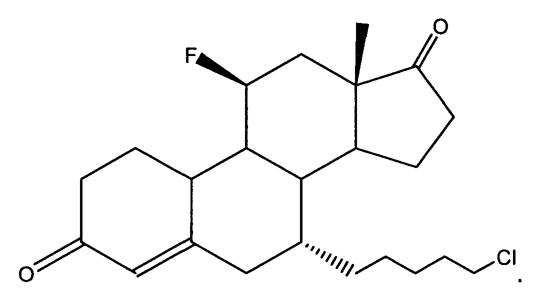

29. The process according to any of the preceding claims, wherein the compound of the general formula **II** has the following chemical structure

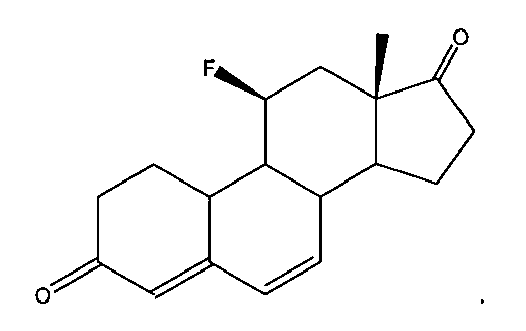

30. A process for the manufacture of a compound of the general formula **III** 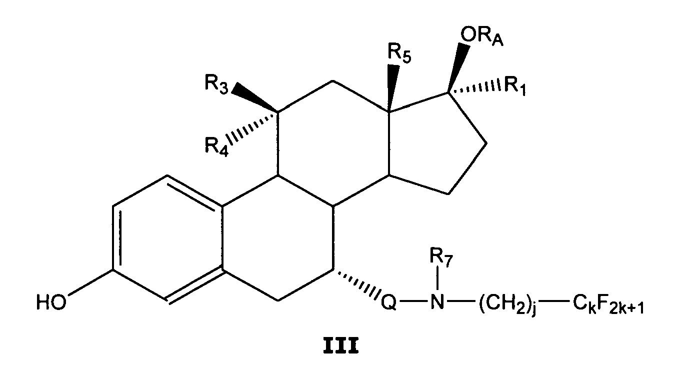 wherein
R₁ is selected from the group consisting of H, C₁-C₆-alkyl, C₂-C₆-alkenyl and C₂-C₆-alkinyl;
R_{A} is H, C₁-C₆-alkyl or C₁-C₆-alkanoyl;
R₃ and R₄ are independently selected from the group consisting of H, F and OR_{B} where R_{B} is H or an oxygen protecting group;
R₅ is CH₃ or CH₂-CH₃;
Q is an alkyl chain having at least 3 carbon atoms, an alkenyl chain having at least 3 carbon atoms, or an alkynyl chain having at least 3 carbon atoms;
R₇ is H or C₁-C₄-alkyl;
j is an integer in the range of from 1 to 10; and
k is an integer in the range of from 1 to 5;
said process comprising the steps of
(i) reacting a compound of the general formula **IIa** 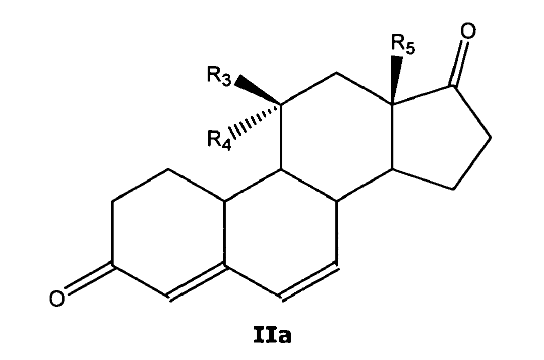 wherein R₃, R₄ and R₅ are as defined above, with a Grignard reagent selected from the group consisting of a magnesium-halide alkyl halide having at least 3 carbon atoms, a magnesium-halide alkenyl halide having at least 3 carbon atoms, and a magnesium-halide alkynyl halide having at least 3 carbon atoms, in the presence of a catalyst system comprising CuI, to obtain a compound of the general formula **Ia** 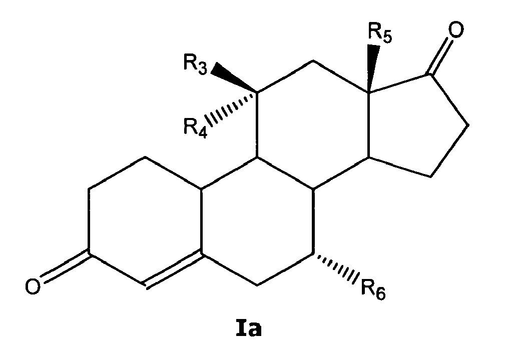 wherein R₃, R₄ and R₅ are as defined above, and R₆ is as defined in any of claims 1-29; and
(ii) converting the compound of the general formula **Ia** to the compound of the general formula III.

31. The process according to claim 30, wherein step (ii) comprises the following steps
(iia) converting a compound of the general formula **Ia** to a compound of the general formula **IV** 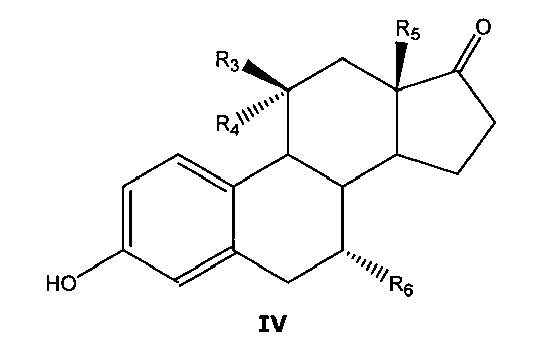
(iib) converting a compound of the general formula **IV** to a compound of the general formula **V** 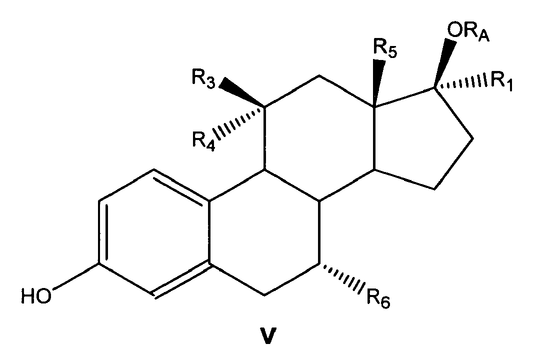
(iic) converting a compound of the general formula **V** to the compound of the general formula **III**,
wherein R_{A}, R₁, R₃, R₄, R₅ and R₆ are as defined in claim 30.

32. The process according to any of claims 31, wherein step (iic) is conducted by reacting a compound of the general formula **V** with a compound of the general formula **VI**
NHR₇-(CH₂)ⱼ-CₖF₂ₖ₊₁ VI
wherein
R₇ is H or C₁-C₄-alkyl;
j is an integer in the range of from 1 to 10; and
k is an integer in the range of from 1 to 5.

33. The process according to any of claims 30-32, wherein R₇ is CH₃.

34. The process according to any of claims 30-33, wherein Q is selected from the group consisting of an alkyl chain having at the most 10 carbon atoms, an alkenyl chain having at the most 10 carbon atoms or an alkynyl chain having at the most 10 carbon atoms.

35. The process according to claim 34, wherein Q is (CH₂)ᵢ and wherein i is an integer in the range of from 3 to 10, such as an integer in the range of from 3 to 7, e.g. in integer in the range of from 4 to 6, in particular 5.

36. The process according to any of claims 34-35, wherein j is an integer in the range of from 5 to 9, such as 5, 6, 7, 8 or 9, e.g. 6, 7 or 8, preferably 7.

37. The process according to any of claims 34-36, wherein k is 1, 2 or 3, preferably 2.

38. The process according to any of claims 30-37, wherein R_{A} is H.

39. The process according to any of claims 30-38, wherein R₁ is C₁-C₆-alkyl, preferably CH₃.

40. The process according to any of claims 30-39, wherein R₃ is F.

41. The process according to any of claims 30-40, wherein R₄ is H.

42. The process according to any of claims 30-41, wherein R₅ is -CH₃.

43. The process according to any of claims 30-42, wherein step (i) is conducted as defined in any of claims 1-29.

44. The process according to any of claims 30-43, wherein the compound of the general formula **VI** has the following chemical structure NH(CH₃)-(CH₂)₇-CF₂-CF₃.

45. The process according to any of claims 30-44, wherein the compound of the general formula **III** has the following chemical structure
